(19) Europäisches Patentamt
European Patent Office
Office européen des brevets

(11) **EP 2 819 659 B1**

(12) **EUROPÄISCHE PATENTSCHRIFT**

(45) Veröffentlichungstag und Bekanntmachung des Hinweises auf die Patenterteilung:
**30.05.2018 Patentblatt 2018/22**

(21) Anmeldenummer: **13703735.4**

(22) Anmeldetag: **11.02.2013**

(51) Int Cl.:
*A61K 9/51* (2006.01)     *A61K 31/4375* (2006.01)
*A61K 31/573* (2006.01)

(86) Internationale Anmeldenummer:
**PCT/EP2013/000396**

(87) Internationale Veröffentlichungsnummer:
**WO 2013/127490 (06.09.2013 Gazette 2013/36)**

(54) **VERFAHREN ZUR HERSTELLUNG WIRKSTOFFBELADENER NANOPARTIKEL**

PROCESS FOR PRODUCING NANOPARTICLES LADEN WITH ACTIVE INGREDIENT

PROCÉDÉ DE FABRICATION DE NANOPARTICULES CHARGÉES D'UNE SUBSTANCE ACTIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.02.2012 DE 102012004099**

(43) Veröffentlichungstag der Anmeldung:
**07.01.2015 Patentblatt 2015/02**

(73) Patentinhaber: **Merck Patent GmbH**
**64293 Darmstadt (DE)**

(72) Erfinder:
• **WEIGANDT, Markus**
**68259 Mannheim (DE)**
• **VOSS, Senta**
**55131 Mainz (DE)**
• **MILLER, Tobias**
**Atlanta, GA 30308 (US)**
• **GOEPFERICH, Achim**
**93161 Sinzing (DE)**

(56) Entgegenhaltungen:
• LETCHFORD ET AL: "A review of the formation and classification of amphiphilic block copolymer nanoparticulate structures: micelles, nanospheres, nanocapsules and polymersomes", EUROPEAN JOURNAL OF PHARMACEUTICS AND BIOPHARMACEUTICS, ELSEVIER SCIENCE PUBLISHERS B.V., AMSTERDAM, NL, Bd. 65, Nr. 3, 28. Februar 2007 (2007-02-28), Seiten 259-269, XP005914364, ISSN: 0939-6411, DOI: 10.1016/J.EJPB.2006.11.009
• LAMPRECHT A ET AL: "Design of rolipram-loaded nanoparticles: comparison of two preparation methods", JOURNAL OF CONTROLLED RELEASE, ELSEVIER, AMSTERDAM, NL, Bd. 71, Nr. 3, 28. April 2001 (2001-04-28), Seiten 297-306, XP004234527, ISSN: 0168-3659, DOI: 10.1016/S0168-3659(01)00230-9
• ELKHARRAZ K ET AL: "Paclitaxel-loaded microparticles and implants for the treatment of brain cancer: Preparation and physicochemical characterization", INTERNATIONAL JOURNAL OF PHARMACEUTICS, ELSEVIER BV, NL, Bd. 314, Nr. 2, 18. Mai 2006 (2006-05-18), Seiten 127-136, XP027972319, ISSN: 0378-5173 [gefunden am 2006-05-18]

**EP 2 819 659 B1**

**Beschreibung**

Die vorliegende Erfindung betrifft ein neues Verfahren zur Herstellung von mit Wirkstoff/en beladenen Nanopartikeln sowie deren Verwendung als Arzneimittel.

Nanopartikel sind ein innovativer Ansatz zur kontrollierten Freisetzung von pharmazeutischen Wirkstoffen. Insbesondere polymerbasierte Nanopartikel fanden in den letzten Jahrzehnten großes Interesse. Bei diesen Systemen wird/werden der/die Wirkstoff/e in eine Polymermatrix eingebettet und wird/werden je nach Beschaffenheit der Matrix kontrolliert freigesetzt. Wesentliche Vorteile solcher Systeme sind: 1) Löslichkeitserhöhung von hydrophoben Wirkstoffen, 2) Senkung von unerwünschten Nebenwirkungen durch zielspezifische Freisetzung, 3) Kontrolle der Wirkstoffpharmakokinetik durch den Wirkstoffträger sowie 4) Verhinderung von vorzeitigem Abbau der Wirkstoffe nach Injektion.

Durch den Polymeranteil in den Nanopartikeln erhöht sich gegenüber der alleinigen Verabreichung des Wirkstoffs die für dessen therapeutische Verabreichung erforderliche Gesamtmenge. Wünschenswert sind daher Nanopartikel mit einer möglichst hohen Wirkstoffbeladung, d.h. einem möglichst hohen Verhältnis an Wirkstoff gegenüber dem Hilfsstoff.

Hilfsstoffe für Arzneimittel müssen hohen Anforderungen im Hinblick auf deren physiologische Unbedenklichkeit und Qualität entsprechen, die in aufwändigen Versuchen gegenüber den zuständigen Zulassungsbehörden nachzuweisen sind. Als Hilfsstoffe für die Entwicklung von Nanopartikeln wurden und werden daher bevorzugt solche Hilfsstoffe verwendet, die zur Verwendung in Arzneimitteln bereits zugelassen sind. Beispiele für in Nanopartikeln infrage kommende bewährte Hilfsstoffe sind Polymilchsäure, Polymilchsäure-co-glykolsäure oder Polycaprolacton.

Nachteilhaft zeigen die bewährten Hilfsstoffe aber oft keine ausgeprägte Kompatibilität zum Wirkstoff und führen daher zu Systemen, die nur gering beladen werden können.

Ein Weg zur Verbesserung der Hilfsstoff-Wirkstoffkompatibilität ist die Verwendung von neuartigen Polymeren. Aufgrund der bereits erwähnten hohen Anforderungen an solche Hilfsstoffe ist die Entwicklung von Nanopartikeln mit solchen (noch nicht zugelassenen) Hilfsstoffen mit einem sehr hohen Aufwand an Zeit und Kosten verbunden. Hinzu kommt zumeist weiterhin, dass die neuartigen Hilfsstoffe auch nicht in der für Arzneimittel erforderlichen Qualität (GMP-Qualität) verfügbar sind.

Nanopartikel auf Grundlage von Polymeren können mit verschiedenen Verfahren hergestellt werden. Die Herstellverfahrens sind:

1. Lösungsmittel-Evaporations-Methoden

a. Lösungsmittel/Nicht-Lösungsmittel-Verfahren (auch bekannt als O/W-Emulsionsverfahren)

Polymer und Wirkstoff werden in einem mit Wasser nicht mischbaren organischen Lösungsmittel, insbesondere Dichlormethan, gelöst und unter konstanter Bewegung/Rühren in eine wässrige Phase eingeleitet. Aus der hierbei entstehenden Emulsion wird anschließend das organische Lösungsmittel entweder unter Vakuum oder bei Normaldruck entfernt (siehe zum Beispiel V.P.Sant, D.Smith, and J.C.Leroux. Enhancement of oral bioavailability of poorly water-soluble drugs by poly(ethylene glycol)-block-poly(alkyl acrylate-co-methacrylic acid) self-assemblies. J Control Release. 104:289-300 (2005)). Während dieses Prozesses entstehen die beladenen Nanopartikel.

b. Multiple Emulsionsverfahren (W/O/W-Verfahren)

W/O/W-Emulsionstechniken eignen sich besonders für die Herstellung von Nanopartikeln mit eher hydrophilen und dadurch wasserlöslichen Wirkstoffen. Das Polymer wird dabei in einem nicht-wassermischbaren Lösungsmittel gelöst (z.B. Dichlormethan) und mit einer wässrigen Phase, die den gelösten Wirkstoff enthält, vereinigt. Die vereinigten Phasen werden homogenisiert (z.B. durch Rühren oder Ultraschallbehandlung), wobei eine W/O-Emulsion erhalten wird. Die W/O-Emulsion wird dann in eine wässrige Phase injiziert, die einen zusätzlichen Emulgator als Stabilisator enthält. Als anschließenden letzten Schritt wird wieder das Lösungsmittel unter Vakuum oder Normaldruck entfernt (K.Avgoustakis, A.Beletsi, Z.Panagi, P.Klepetsanis, A.G.Karydas, and D.S.Ithakissios. PLGA-mPEG nanoparticles of cisplatin: in vitro nanoparticle degradation, in vitro drug release and in vivo drug residence in blood properties; J Control Release. 79:123-135 (2002); C.X.Song, V.Labhasetwar, H.Murphy, X.Qu, W.R.Humphrey, R.J.Shebuski, and R.J.Levy. Formulation and characterization of biodegradable nanoparticles for intravascular local drug delivery. Journal of Controlled Release. 43:197-212 (1997)).

c. Co-Lösungsmittel-Evaporation

Bei der Methode werden Wirkstoff und Polymer in einem organischen Lösungsmittel gemischt und in die wässrige Phase injiziert. Das organische Lösungsmittel wird im Vakuum oder bei Normaldruck entfernt. Im Unterschied zu den Emulsionsmethoden ist das verwendete Lösungsmittel hier vollständig mit Wasser mischbar, so dass keine Emulsionen entstehen.

2. Dialyse

a. Direkte Dialyse

Bei dem Verfahren werden Wirkstoff sowie Polymer in einem mit Wasser mischbaren organischen Lösungsmittel gelöst und in eine Dialysevorrichtung eingebracht. Die Dialyse kann gegen Wasser oder Puffer erfolgen. Die Herstellung der Nanopartikel erfolgt durch langsamen, stetigen Austausch des Lösungsmittels folgend dem Konzentrationsgradient von der inneren und äußeren Phase. Bemerkenswert bleibt, dass die verwendeten Dialysemembranen zwar durchlässig für kleine Moleküle sind (Wirkstoff), das Polymer aufgrund der Ausschlussgrößen aber in der inneren Phase verbleibt (H.J.Jeon, J.I.Jeong, M.K.Jang, Y.H.Park, and J.W.Nah. Effect of solvent on the preparation of surfactant-free poly(DL-lactide-co-glycolide) nanoparticles and norfloxacin release characteristics. International Journal of Pharmaceutics. 207:99-108 (2000)).

3. Film-Hydratationsmethoden

Dieses Verfahren ist ein Standard zur Herstellung von liposomalen Formulierungen. Dabei wird das Lipid oder Polymer in einem organischen Lösungsmittel gelöst und unter Vakuum evaporiert. Der sich im Glasgerät bildende Film wird anschließend mit Puffer, Wirkstofflösung oder Wasser rekonstituiert. Nachteilig ist, dass der resultierende Polymer-Wirkstofffilm nicht oder nur teilweise redispergierbar sein kann (A.Richter, C.Olbrich, M.Krause, J.Hoffmann, and T.Kissel. Polymeric micelles for parenteral delivery of Sagopilone: physicochemical characterization, novel formulation approaches and their toxicity assessment in vitro as well as in vivo. Eur J Pharm Biopharm. 75:80-89 (2010)). Gelingt die Resdispergierung des Films doch, folgt dem Verfahren nach Herstellung der Rohpartikel meist ein Schritt der Größenklassifizierung (Membranextrusion, Ultraschallbehandlung) (E.Blanco, E.A.Bey, Y.Dong, B.D.Weinberg, D.M.Sutton, D.A.Boothman, and J.Gao. Beta-lapachone-containing PEG-PLA polymer micelles as novel nanotherapeutics against NQO1-overexpressing tumor cells. J Control Release. 122:365-374 (2007); Richter et al. wie oben zitiert).

Eine Übersichtsarbeit über die Bildung und Klassifizierung von amphiphilen Block-Copolymer-nanopartikulären Strukturen erwähnt Verfahren zur Herstellung von Nanosphären wie Lösungsmittelentfernung (solvent displacement) und Nanopräzipitation, ohne auf deren Einzelheiten einzugehen (K. Lechtford et al. A a review about formation and classification of amphiphilic block copolymer nanoparticulate structures:micelles, nanospheres, nanocapsules and polymersomes. Eur J Pharm Biopharm 65 (2007) 259-269).

Lamprecht A. et al. beschreibt eine Dialyse von Nanopartikeln gegen reines Wasser, um Hilfsstoffe und nicht verkapselten Wirkstoff zu entfernen (Alf Lamprecht A. et al. Design of rolipram-loaded nanoparticles: comparison of two preparation methods. J Contr Release 71 (2001) 297-306) K. Elkharraz E. et al beschreibt die Herstellung von Wirkstoff-freien und mit Paclitaxel beladenen PLGA-Mikropartikeln mit einem Öl in Wasser (O/W) Lösungsmittel-Extraktions- / Evaporationsmethode (Paclitaxel-loaded microparticles and implants for the treatment of brain cancer: Preparation and physicochemical characterization. Int J Pharm 314 (2006) 127-136).

Herstellung von Nanopartikeln mit bewährten Polymeren mittels den vorstehend beschriebenen Verfahren führt oft zu einer für deren therapeutische Anwendung unzureichenden Wirkstoffbeladung. Wünschenswert wäre die Bereitstellung eines Verfahrens zur Herstellung von Nanopartikeln, das mit herkömmlichen Hilfsstoffen trotz der hierbei gegebenen Hilfsstoff-Wirkstoff-Kompatibilitätsprobleme die Herstellung von Nanopartikeln mit hoher Wirkstoffbeladung erlaubt.

Die bekannten Verfahren führen aufgrund der Einbettung des Wirkstoffs in das Polymer auch zu Nanopartikeln, aus denen der Wirkstoff erst mit einer gewissen zeitlichen Verzögerung (lag time) freigesetzt wird. Dies hat zur Folge dass nach Verabreichung der Nanopartikel therapeutische Wirkstoffspiegel erst mit einer zeitlichen Verzögerung erreicht werden, so dass für die Erzielung eines schnellen Wirkungseintritts die zusätzliche Verabreichung des Wirkstoffs in einer schnell verfügbaren Form erforderlich ist. Wünschenswert wären daher weiterhin Nanopartikel, die direkt nach Verabreichung eine Menge an Wirkstoff freisetzen, so dass der Wirkstoff bereits vor der verzögerten Wirkstofffreisetzung aus den Polymeren in therapeutischer Menge zur Verfügung gestellt wird.

Es war daher Aufgabe der vorliegenden Erfindung ein Verfahren zur Herstellung von Nanopartikeln bereitzustellen, dass nicht mit den vorstehend angeführten Nachteilen der bestehenden Herstellverfahren behaftet ist. Das Verfahren sollte insbesondere die Bereitstellung von Nanopartikeln ermöglichen, die gegenüber mit herkömmlichen Verfahren und herkömmlichen Polymeren hergestellten Nanopartikeln höhere Wirkstoffbeladungen aufweisen. Weiterhin sollten die hergestellten Nanopartikel bereits direkt nach ihrer Verabreichung eine Initialdosis des Wirkstoffs freisetzen und so zu einem zeitnahen Wirkungseintritt führen.

Diese Aufgaben konnten mit einem Verfahren zur Herstellung von Nanopartikeln gelöst werden, dass die folgenden Schritte umfasst: (a) Lösen von mindestens einem Wirkstoff und mindestens einem Polymer in einem organischen Lösungsmittel, (b) Mischen der in Schritt (a) hergestellten Lösung mit einer wässrigen Phase, (c) Evaporieren des organischen Lösungsmittels, (d) Aufreinigen der in Schritt (c) erhaltenen, mit Wirkstoff beladenen Nanopartikel mittels Dialyse gegen den gleichen Wirkstoff enthaltende wässrige Dialyselösung. Gegenstand der Erfindung ist daher ein Verfahren zur Herstellung von Nanopartikeln umfassend die Schritte (a) Lösen von mindestens einem Wirkstoff und mindestens einem Polymer in einem organischen Lösungsmittel, (b) Mischen der in Schritt (a) hergestellten Lösung mit einer wässrigen Phase, (c) Evaporieren des organischen Lösungsmittels, (d) Aufreinigen der in Schritt (c) erhaltenen, mit Wirkstoff beladenen Nanopartikel mittels Dialyse gegen den gleichen Wirkstoff enthaltende wässrige Dialyselösung.

Die beim Lösen des Wirkstoffs und des Polymers in einem organischen Lösungsmittel gemäß Schritt (a) entstehende Lösung wird vor- und nachstehend auch als organische Phase bezeichnet.

Als wässrige Phase kann Wasser eingesetzt werden, in dem wasserlösliche Stoffe, insbesondere Salze, wie zum Beispiel Puffersalze, Säuren oder Basen gelöst sind. Das Mischen der gemäß Schritt (a) hergestellten Lösung mit der wässrigen Phase kann erfolgen, indem die gemäß Schritt (a) hergestellte Lösung zu der wässrigen Phase gegeben wird oder die wässrige Phase zu der gemäß Schritt (a) hergestellten Lösung gegeben wird. Vorzugsweise erfolgt die Zugabe der einen Lösung zu der anderen unter stetigem Rühren oder Schütteln und ggf. unter Einsatz von Ultraschall. Zweckmäßigerweise wird beim Mischen die Phase mit dem kleineren Volumen der Phase mit dem größeren Volumen zugefügt, aber auch das umgekehrte Verfahren ist möglich.

Die wässrige Phase besteht aus einem wässrigen Lösungsmittel. Ein "wässriges Lösungsmittel" im Sinne der Erfindung ist Wasser, das hierin gelöste Stoffe enthalten kann, insbesondere, Elektrolyte, wie zum Beispiel Salze, Säuren oder Basen.

Die Entfernung des organischen Lösungsmittels kann durch Verdampfung (Evaporieren) unter Normalbedingungen, d.h. bei Raumtemperatur und Normaldruck erfolgen und kann durch Erhöhung der Temperatur und/oder Verminderung des Drucks, d.h. durch Senkung des Drucks auf einen Wert unterhalb des Luftdrucks, beschleunigt werden. Bevorzugt erfolgt die Evaporation bei erhöhter Temperatur, besonders bevorzugt bei 30 bis 60°C und/oder bei reduziertem Druck, bevorzugt bei $10^{-4}$ bis 80 mbar. Zweckmäßig kann die Evaporation zum Beispiel mit einem Rotationsverdampfer erfolgen.

Die Dialyse kann mit den üblichen, dem Fachmann bekannten Dialysevorrichtungen, beispielsweise mit einem im Labor üblichen Dialyseschlauch erfolgen. Zweckmäßigerweise ist die Porengröße der Dialysemembran so gewählt, dass das organische Lösungsmittel und der Wirkstoff die Dialysemembran frei passieren können, das Polymer jedoch nicht. Die obere Ausschlussmasse (Molecular Weigh Cutoff (MWCO)) einer geeigneten Dialysemembran liegt daher vorzugsweise oberhalb der Molekülmasse des Wirkstoffs und des Lösungsmittels aber unterhalb der Molekülmasse der kleinsten im Polymer enthaltenen Polymermoleküle. Beispielsweise kann bei einer unteren Molekülmasse des Polymers von 15 kDa und einer molaren Masse des Wirkstoffs von zum Beispiel 300 Da eine Dialysemembran mit einem MWCO unterhalb 5 kDa sowie oberhalb von 1.5 kDa verwendet werden. Üblich und kommerziell erhältlich sind in diesem Beispiel Dialysemembranen mit einem MWCO von 3.5 kDa oder 5 kDa.

Enthält die Dialyselösung keinen Wirkstoff kommt es durch die Dialyse aufgrund der Konzentrationsunterschiede zwischen der die Nanopartikel enthaltenden Seite und der die Dialyselösung enthaltenden Seite zu einer Verminderung von Wirkstoff von der die Nanopartikel enthaltenden Seite. Infolge des Verlustes an Wirkstoff in dem Lösungsmittel, das die Nanopartikel umgibt, ergibt sich ein starkes Gefälle der Konzentration an Wirkstoff von den Nanopartikeln gegenüber diesem Lösungsmittel, mit der Folge, dass Wirkstoff aus den Nanopartikeln austreten kann und dann aufgrund des Konzentrationsgradienten zur Dialyselösung übertritt und abtransportiert wird. Durch die erfindungsgemäße Verwendung von Dialyselösung, in der der Wirkstoff gelöst ist, wird dieses Konzentrationsgefälle zumindest vermindert und damit dem Wirkstoffverlust aus den Nanopartikeln entgegengewirkt. Der Wirkstoff kann in der Dialyselösung in allen Konzentrationen bis zu dessen maximalen Löslichkeit (Sättigungslöslichkeit) in der Dialyselösung enthalten sein, bevorzugt ist die Dialyselösung mit Wirkstoff gesättigt.

Die Wirkstoffbeladung der Nanopartikel kann auch durch Diffusion und Adhäsion an die eingesetzten Membranen sinken. Nach einer bevorzugten Ausführungsform wird daher die Dialysemembran vor Durchführung der Dialyse mit Wirkstoff enthaltender Dialyselösung, bevorzugt mit einer mit Wirkstoff gesättigten Dialyselösung, in Kontakt gebracht, so dass sich die Membran entsprechend der in der Dialyselösung enthaltenen Wirkstoffkonzentration mit Wirkstoff anreichert. Nach einer besonders bevorzugten Ausführungsform der Erfindung wird die Dialyse gegen mit Wirkstoff gesättigter Dialyselösung und mit einer mit Wirkstoff gesättigten Dialysemembran durchgeführt.

Als Folge des in der Dialyselösung enthaltenen Wirkstoffs weisen die gemäß dem vorliegenden Verfahren hergestellten Nanopartikel weiterhin eine Menge an Wirkstoff auf, die an den Nanopartikeln adsorbiert ist. Dieser Wirkstoffanteil steht vorteilhaft direkt bei Verabreichung an den Patienten als Initialdosis zur Verfügung.

Nach einer vorteilhaften Ausführungsform der Erfindung enthält die Dialyselösung neben dem Wirkstoff auch gelöste Stoffe, insbesondere Elektrolyte, besonders bevorzugt Puffer und/oder Salze, die auch in der für die Verabreichung der Nanopartikel vorgesehenen Formulierung enthalten sein sollen. Aufgrund des Flüssigkeitsaustauschs infolge der Dialyse können auf diesem Weg vorteilhaft und in einfacher Weise Nanopartikel erhalten werden, die bereits in einem für die Verabreichung an den Patienten geeigneten Lösungsmittel enthalten sind. Vor Verabreichung an den Patienten ist somit nur noch die Keimfreiheit sicherzustellen, was beispielsweise und in einfacher Weise mittels Sterilfiltration erfolgen kann. Alternativ kann auch das gesamte Herstellverfahren unter aseptischen Bedingungen gearbeitet werden, so dass eine nachträgliche Sterilisation nicht erforderlich ist.

Nach der Dialyse liegen die Nanopartikel in dem wässrigen Lösungsmittel vor. Sind, wie vorstehend beschrieben, die Nanopartikel in einem zu für die Verabreichung an den Patienten geeigneten Lösungsmittel erhalten, steht bei Verabreichung an den Patienten neben an die Nanopartikel adsorbiertem Wirkstoff auch im Lösungsmittel gelöster Wirkstoff als Initialdosis zur Verfügung.

Werden den Nanopartikeln, beispielsweise zur Stabilisierung, das wässrige Lösungsmittel entzogen, was zum Beispiel bevorzugt mittels Gefriertrocknung oder auch mittels Sprühtrocknung erfolgen kann, präzipitiert im Lösungsmittel gelöster Wirkstoff während des Wasserentzugs zumindest zum Teil auf den Nanopartikeln, so dass dieser ebenfalls bei Verabreichung an den Patienten als Initialdosis zur Verfügung steht.

In dem erfindungsgemäßen Verfahren werden bevorzugt Wirkstoffe mit einer geringen Sättigungslöslichkeit in Wasser, vorzugsweise mit einer Sättigungslöslichkeit < 200 $\mu$g/ml, besonders bevorzugt mit einer Sättigungslöslichkeit <100 $\mu$g/ml, eingesetzt (jeweils gemessen bei 25 °C). Gegenstand der Erfindung ist daher auch ein Verfahren, das dadurch gekennzeichnet ist, dass der Wirkstoff in Wasser eine Sättigungslöslichkeit < 200 $\mu$g/ml, bevorzugt eine Sättigungslöslichkeit <100 $\mu$g/ml, jeweils gemessen bei 25°C, aufweist.

Als Wirkstoffe besonders bevorzugt sind Wirkstoffe ausgewählt aus der Gruppe bestehend aus Chemotherapeutika, insbesondere Taxol, Camptothecin, Platinkomplexe oder N-Lost-Verbindungen, Antirheumatika, wie zum Beispiel Glucocorticoide, insbesondere Dexamethason, Mometason, Beclomethason oder Prednisolon, Antiinfektiva, wie zum Beispiel HIV-Therapeutika, insbesondere Ritonavir, sowie Antimykotika, insbesondere Ketoconazol, Itraconazol, Griseofulvin, Lipidsenker, wie zum Beispiel Fenofibrat, Antioxidatien und Vitamine, wie zum Beispiel Tocopherol, Retinolsäure-, Cholecalciferol, Antibiotika, wie zum Beispiel Vancomycin oder Teicomycin, zusätzlich Cholesterol sowie Fettsäuren. Gegenstand der Erfindung ist daher weiterhin ein Verfahren, das dadurch gekennzeichnet ist, dass als Wirkstoff ein Wirkstoff verwendet wird, das ausgewählt ist aus der Gruppe bestehend aus Chemotherapeutika, insbesondere Taxol, Camptothecin, Platinkomplexe oder N-Lost-Verbindungen, Antirheumatika, wie zum Beispiel Glucocorticoide, insbesondere Dexamethason, Mometason, Beclomethason oder Prednisolon, Antiinfektiva, wie zum Beispiel HIV-Therapeutika, insbesondere Ritonavir, sowie Antimykotika, insbesondere Ketoconazol, Itraconazol, Griseofulvin, Lipidsenker, wie zum Beispiel Fenofibrat, Antioxidatien und Vitamine, wie zum Beispiel Tocopherol, Retinolsäure-, Cholecalciferol, Antibiotika, wie zum Beispiel Vancomycin oder Teicomycin, zusätzlich Cholesterol sowie Fettsäuren.

Nach einer vorteilhaften Ausführungsform der Erfindung wird in dem Verfahren als Polymer ein amphiphiles Polymer eingesetzt. Gegenstand der Erfindung ist daher auch das erfindungsgemäße Verfahren, das dadurch gekennzeichnet ist, dass als Polymer ein amphiphiles Polymer eingesetzt wird. Amphiphile Polymere sind aus einem hydrophilen ("wasserliebenden") und einem hydrophoben ("wasserhassenden") Teilen aufgebaut. Aufgrund dieser Struktur lagern sich amphiphile Polymere in heterogenen Gemischen aus Wasser und mit Wasser nicht mischbaren Lösungsmitteln, insbesondere organischen Lösungsmitteln, wie zum Beispiel Dichlormethan, bevorzugt an den Grenzflächen zwischen der wässrigen und der organischen Phase, an.

Nach einer besonders vorteilhaften Ausführungsform der Erfindung werden als amphiphile Polymere Blockcopolymere verwendet. Gegenstand der Erfindung ist daher auch ein Verfahren, das dadurch gekennzeichnet ist, dass als Polymer ein Blockcopolymer eingesetzt wird. Blockcopolymere bestehen aus einem oder mehreren, auch verschiedenen Blöcken aus einer hydrophilen Komponente a) und einer hydrophobe Komponente b), wobei die einzelnen Blöcke gleiche Monomere mit gleicher oder unterschiedlicher Kettenlänge oder verschiedene Monomere enthalten können. Die Komponenten a) und b) können gleichzeitig oder unabhängig voneinander linear oder verzweigt, kamm- oder sternförmig sein. Komponente b) kann auch ein quervernetztes Polymer sein.

Als hydrophobe Komponente b) besonders geeignet sind bioabbaubare Polymere wie zum Beispiel Polyester, Poly-$\varepsilon$-caprolacton, Poly-$\alpha$-hydroxyester, Poly-ß-hydroxyester, Polyanhydrid, Polyamid, Polyphospazen, Polydioxanon, Polyäpfelsäure, Polyweinsäure, Polyorthoester, Polycarbonat, Polysaccharid, Peptid und Protein.

Als hydrophile Komponente a) ist aufgebaut aus mindestens bifunktionalen und vorzugsweise wasserlöslichen Bausteinen, Beispiele für geeignete Polymere sind Polyethylenglykole, Polyacrylamide, Polyvinylalkohol, Polysaccharide (zum Beispiel modifizierte Cellulosen und Stärken), Alginate, Peptide und Proteine.

Erfindungsgemäß einsetzbare Blockcopolymere können als hydrophile Komponente zum Beispiel Polyethylenglykol, Polypropylenglykol, Polybutylenglykol, Polyacrylamid, Polyvinylalkohol, Polysaccharid oder ein Copolymer hiervon, bevorzugt Polyethylenglykol/Polypropylenglykol-Copolymer, Polyethylenglykol/Polypropylenglykol/Polyethylenglykol-Copolymer, und als hydrophobe Komponente Polymilchsäure, Polyglykolsäure, Polyhydroxybuttersäure, Polyhydroxyvaleriansäure, oder ein Copolymer hiervon, bevorzugt Polymilch-co-glykolsäure, weiterhin Polyacrylsäure , insbesondere Hydroxypropylethylacrylsäure oder Hydroxypropylmethylacrylsäure, Polysiloxan , insbesondere Copolymere mit Acrylsäure, Polystyren oder ein Copolymer hiervon, insbesondere mit Polymilchsäure und Polyglykolsäure enthalten. Gegenstand der Erfindung ist daher auch ein Verfahren, das dadurch gekennzeichnet ist, dass das Blockcopolymer als hydrophile Komponente Polyethylenglykol, Polypropylenglykol, Polybutylenglykol, Polyacrylamid, Polyvinylalkohol, Polysaccharid oder ein Copolymer hiervon, bevorzugt Polyethyleng lykol/Polypropylenglykol-Copolymer, Polyethylenglykol/Polypropylenglykol/Polyethylenglykol-Copolymer, und als hydrophobe Komponente Polymilchsäure, Polyglykolsäure, Polyhydroxybuttersäure, Polyhydroxyvaleriansäure, oder ein Copolymer hiervon, bevorzugt Polymilch-co-glykolsäure, weiterhin Polyacrylsäure , insbesondere Hydroxypropylethylacrylsäure oder Hydroxypropylmethylacrylsäure, Polysiloxan , insbesondere Copolymere mit Acrylsäure, Polystyren oder ein Copolymer hiervon, insbesondere mit Polymilchsäure und Polyglykolsäure enthält.

Nach einer vorteilhaften Ausbildung der Erfindung werden als als Blockcopolymere Polyethylenglykol-Polymilchsäure, Polyethylenglykol-Polyglykolsäure, Polyethylenglykol-Polymilchsäure-co-glykolsäure, Polyethylenglykol-Polyhydroxyvaleriansäure, Polyethylenglykol-Polysiloxan, Polyethylenglykol-Polysiloxan-co-acrylsäure, Polyethylenglykol-Polymethylmethacrylsäure, Polyethylenglykol-Polymethyl-ethacrylsäure, Polyethylenglykol-Polyisoprylacrylsäure, Polyethylenglykol-Polystyren eingesetzt. Gegenstand der Erfindung ist daher auch ein Verfahren, das dadurch gekennzeichnet ist, dass als Blockcopolymere Polyethylenglykol-Polymilchsäure, Polyethylenglykol-Polyglykolsäure, Polyethylenglykol-Polymilchsäure-co-glykolsäure, Polyethylenglykol-Polyhydroxyvaleriansäure, Polyethylenglykol-Polysiloxan, Polyethylenglykol-Polysiloxan-co-acrylsäure, Polyethylenglykol-Polymethylmethacrylsäure, Polyethylenglykol-Polymethyl-ethacrylsäure, Polyethylenglykol-Polyisoprylacrylsäure, Polyethylenglykol-Polystyren eingesetzt wird.

Nach einer weiteren vorteilhaften Ausführungsform wird in dem erfindungsgemäßen Verfahren als organisches Lösungsmittel ein Lösungsmittel eingesetzt, das mit Wasser zumindest teilweise mischbar, bevorzugt vollständig mischbar ist. Gegenstand der Erfindung ist daher auch ein Verfahren, das dadurch gekennzeichnet ist, dass als organisches Lösungsmittel ein Lösungsmittel verwendet wird, das mit Wasser zumindest teilweise mischbar, bevorzugt vollständig mischbar ist.

Im Sinne der Erfindung ist ein mit Wasser zumindest teilweise mischbares Lösungsmittel ein Lösungsmittel, dem bei Raumtemperatur (25 °C) unter Erhaltung einer einheitlichen, homogenen Phase Wasser in einem Volumenverhältnis von mindestens 40/60 v/v (organisches Lösungsmittel/Wasser) zumischbar ist. Wird der unter Erhaltung einer homogenen Phase maximal zumischbare Wasseranteil überschritten kommt es zu einer Phasentrennung zwischen der homogenen organischen und Wasser enthaltenden ersten Phase und einer zweiten, aus Wasser bestehenden Phase. Ein mit Wasser vollständig mischbares organisches Lösungsmittel ist ein organisches Lösungsmittel, dem bei Raumtemperatur (25 °C) unter Erhaltung einer einheitlichen, homogenen Phase Wasser in jedem Volumenverhältnis zumischbar ist.

In dem erfindungsgemäßen Verfahren einsetzbare organische Lösungsmittel sind lineare oder verzweigtkettige Alkohole, vorzugsweise Methanol, Ethanol, Isopropanol, n-Butanol oder tert.-Butanol, Aceton , Dimethylformamid, Tetrahydrofuran oder Dimethylsulfoxid. Gegenstand der Erfindung ist daher auch ein Verfahren, das dadurch gekennzeichnet ist, dass als organisches Lösungsmittel lineare oder verzweigtkettige Alkohole, vorzugsweise Methanol, Ethanol, Isopropanol, n-Butanol oder tert.-Butanol, Aceton , Dimethylformamid, Tetrahydrofuran oder Dimethylsulfoxid eingesetzt wird.

Wirkstoffe, die Säuren oder Basen sind, können erfindungsgemäß bevorzugt zur Erhöhung ihrer Löslichkeit jeweils komplementär mit einer Base oder einer Säure versetzt werden. Ist der Wirkstoff eine Säure, wird diesem also eine Base zugegeben, ist er eine Base erfolgt der Zusatz einer Säure. Die Säure bzw. Base kann in dem Verfahren gemäß Anspruch 1 in Schritt (a) oder in Schritt (b) zugesetzt werden. Gegenstand der Erfindung ist daher auch eine Ausführungsform des erfindungsgemäßen Verfahrens, das dadurch gekennzeichnet ist, dass in Schritt (a) gemäß Anspruch 1 in dem organischen Lösungsmittel neben Polymer und Wirkstoff eine Säure oder eine Base gelöst ist und/oder dass in dem wässrigen Lösungsmittel gemäß Schritt (b) von Anspruch 1 eine Säure oder eine Base gelöst ist.

Geeignete Säuren sind organische Säuren, vorzugsweise Ameisensäure, Essigsäure oder Trifluoressigsäure, oder anorganische Säuren, vorzugsweise Salzsäure, Salpetersäure oder Schwefelsäure, geeignete Basen sind organische Basen, vorzugsweise Dimethylamin oder Trimethylamin oder anorganische Basen, vorzugsweise Natriumhydroxid, Kaliumhydroxid oder Ammoniak. Gegenstand der Erfindung ist daher auch ein Verfahren, das dadurch gekennzeichnet ist, dass die Säure eine organische Säure, vorzugsweise Ameisensäure, Essigsäure oder Trifluoressigsäure, oder eine anorganische Säure, vorzugsweise Salzsäure, Salpetersäure oder Schwefelsäure, und die Base eine organische Base, vorzugsweise Dimethylamin oder Trimethylamin oder eine anorganische Base, vorzugsweise Natriumhydroxid, Kaliumhydroxid oder Ammoniak, ist.

Nach einer vorteilhaften Ausführungsform des erfindungsgemäßen Verfahrens wird das zum Lösen von Wirkstoff und Polymer eingesetzte organische Lösungsmittel wie nachfolgend beschrieben ausgewählt. Hierbei wird in einer Auswahl von mit Wasser zumindest teilweise mischbaren Lösungsmitteln jeweils eine definierte Menge des Wirkstoffs, der in die Formulierung eingebettet werden soll, zugegeben und gelöst. Nach einer zweckmäßigen Ausführungsform der Erfindung enthält die Auswahl an Lösungsmitteln zum Beispiel Alkohole (Methanol, Ethanol, Isopropanol, 1-Propanol, tert-Butanol), Dimethylsulfoxid (DMSO), Dimethylformamid (DMF), Dioxan, Tetrahydrofuran (THF), Acetonitril (ACN) und Aceton. Ist der Wirkstoff eine Säure oder Base werden diese vorzugsweise gemäß der oben beschriebenen Vorgehensweise jeweils mit einer Säure oder einer Base versetzt.

Für die Lösung werden jeweils gleiche Mengen an Wirkstoff in jeweils gleichen Mengen an organischen Lösungsmitteln gelöst. Die Menge an Wirkstoff und organischem Lösungsmittel, die jeweils eingesetzt wird, ist variabel, entscheidend ist, dass das Verhältnis von Wirkstoff zu Lösungsmittel jeweils gleich ist. Für die praktische Umsetzung hat es sich als zweckmäßig erwiesen jeweils 1 mg Wirkstoff in 100 µl organischem Lösungsmittel zu lösen.

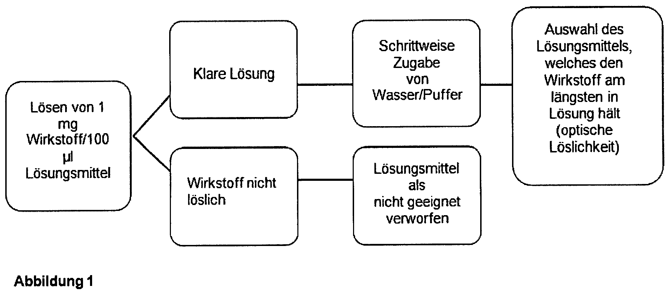

Ist der Wirkstoff in einem organischen Lösungsmittel nicht löslich, ist dieses Lösungsmittel für die Herstellung von Nanopartikeln weniger geeignet. Ergibt sich eine klare Lösung werden schrittweise so lange jeweils gleiche definierte Mengen an wässrigem Lösungsmittel zugesetzt und jeweils gemischt bis nach Zugabe und Mischen eine Präzipitation des Wirkstoffs optisch erkennbar ist (optische Löslichkeit). Die Beurteilung der optischen Löslichkeit erfolgt visuell in einem geeigneten Behältnis, vorzugsweise in einem Glasröhrchen mit geringem Durchmesser, zum Beispiel wie sie in der Gaschromatografie gebräuchlich sind (Durchmesser von 0,5 cm, 3 cm Höhe) bei Kaltlicht, das vorzugweise oben abstrahlt, gegen eine dunkle Oberfläche, vorzugsweise gegen eine schwarze Oberfläche, als Hintergrund. Eine schematische Darstellung der Vorgehensweise ist in Abbildung 1 dargestellt.

Nach einer bevorzugten Ausführungsform der Erfindung wird als wässriges Lösungsmittel ein wässriges Lösungsmittel eingesetzt, dass die gleiche Zusammensetzung wie das wässrige Lösungsmittel, das in dem Verfahren gemäß Schritt (b) nach Anspruch 1 Verwendung findet.

Dasjenige organische Lösungsmittel, welchem der höchste Anteil an wässrigem Lösungsmittel zumischbar ist, ohne dass der Wirkstoff aus der Lösung ausfällt, ist für die Verwendung in dem erfindungsgemäßen Verfahren besonders geeignet. Gegenstand der vorliegenden Erfindung ist daher auch eine Ausführungsform des Verfahrens, dass dadurch gekennzeichnet ist, dass bei der Herstellung der Nanopartikel gemäß Schritt (a) von Anspruch 1 als organisches Lösungsmittel dasjenige organische Lösungsmittel verwendet wird, dem bei Herstellung einer den Wirkstoff in definierter Menge enthaltenden Lösung im Vergleich zu diesen Wirkstoff in jeweils gleicher Menge enthaltenden Lösungen in anderen organischen Lösungsmitteln bei sukzessiver Zumischung von wässrigem Lösungsmittel der höchste Anteil an wässrigem Lösungsmittel zumischbar ist, ohne dass der Wirkstoff aus der Lösung ausfällt.

Durch Verwendung eines organischen Lösungsmittels, dem bei sukzessiver Zumischung von wässrigem Lösungsmittel der höchste Anteil an wässrigem Lösungsmittel zumischbar ist, kann sichergestellt werden, dass Wirkstoff und Polymer bei dem Evaporieren des organischen Lösungsmittels gemäß Schritt (c) von Anspruch 1, im Vergleich zu der Verwendung von anderen Lösungsmitteln, länger in Lösung bleiben.

Die Evaporation des organischen Lösungsmittels gemäß Schritt (c) nach Anspruch 1 aus dem gemäß Schritt (b) hergestellten Lösungsmittelgemisch führt infolge der Verringerung des Anteils an organischem Lösungsmittel in dem Lösungsmittelgemisch zu einer kontinuierlichen Verminderung der Löslichkeit von Wirkstoff und Polymer in dem Lösungsmittelgemisch. Ergibt sich für ein organisches Lösungsmittel in Mischung mit dem wässrigen Lösungsmittel bei der Evaporation bereits bei geringer Abnahme seines Anteils im Lösungsmittelgemisch eine zu geringe Löslichkeit für den Wirkstoff, präzipitiert der Wirkstoff bereits zu einem Zeitpunkt, bei dem das Polymer noch vollständig oder weitgehend gelöst vorliegt und noch keine Nanopartikel gebildet werden. Präzipitiert der Wirkstoff vor Bildung der Nanopartikel kann er nicht mehr in dem Polymer eingeschlossen werden, so dass im Verlauf der weiteren Evaporation Nanopartikel erhalten werden, in denen wenig oder überhaupt kein Wirkstoff eingebettet ist.

Mit Verwendung eines organischen Lösungsmittels, dem bei sukzessiver Zumischung von wässrigem Lösungsmittel der höchste Anteil an wässrigem Lösungsmittel zumischbar ist, kann der Zeitraum, in dem der Wirkstoff während der Evaporation in Lösung vorliegt, verlängert und frühzeitige unerwünschte Wirkstoffpräzipitation vermieden werden. Durch den verlängerten Zeitraum, in dem der Wirkstoff während der Evaporation in Lösung vorliegt, bilden sich bevorzugt zuerst die Nanopartikel, die gleichzeitig einen Teil des Wirkstoffs einschließen. Mit fortschreitender Evaporation reicht der abnehmende Anteil an organischem Lösungsmittel in der Mischung nicht mehr aus, den Wirkstoff durch den Cosolventseffekt in Lösung zu halten. Schließlich verteilt sich der noch nicht eingekapselte Wirkstoff in diesem Prozess in den hydrophoben Kern der Nanopartikel.

Nach einer zweckmäßigen Ausführungsform vorliegenden Erfindung wird das organische Lösungsmittel gemäß nachfolgendem Verfahren ermittelt:

(a) Herstellen von Lösungen des Wirkstoffs mit jeweils gleichem Wirkstoffanteil in verschiedenen organischen Lösungsmitteln,

(b) Zugeben einer jeweils gleichen Menge wässriger Lösung zu jeder der in Schritt (a) hergestellten Lösungen,

(c) Überprüfen ob der Wirkstoff in den Lösungen von Schritt (b) jeweils vollständig gelöst vorliegt,

(d) wiederholtes Durchführen der Schritte (b) und (c) mit den Lösungen, in denen der Wirkstoff gemäß Schritt (c) vollständig gelöst vorliegt, bis der Wirkstoff gemäß Schritt (c) nicht mehr vollständig gelöst vorliegt,

(e) Identifizieren des organischen Lösungsmittels, dem die höchste Menge an wässriger Lösung gemäß Schritt (d) kumulativ zumischbar ist bevor der Wirkstoff nicht mehr vollständig gelöst vorliegt.

Gegenstand der vorliegenden Erfindung ist daher auch eine Ausführungsform des Verfahrens, das dadurch gekennzeichnet ist, dass das organische Lösungsmittel gemäß nachfolgendem Verfahren ermittelt wird:

(a) Herstellen von Lösungen des Wirkstoffs mit jeweils gleichem Wirkstoffanteil in verschiedenen organischen Lösungsmitteln,

(b) Zugeben einer jeweils gleichen Menge wässriger Lösung zu jeder der in Schritt (a) hergestellten Lösungen,

(c) Überprüfen ob der Wirkstoff in den Lösungen von Schritt (b) jeweils vollständig gelöst vorliegt,

(d) wiederholtes Durchführen der Schritte (b) und (c) mit den Lösungen, in denen der Wirkstoff gemäß Schritt (c) vollständig gelöst vorliegt, bis der Wirkstoff gemäß Schritt (c) nicht mehr vollständig gelöst vorliegt,

(e) Identifizieren des organischen Lösungsmittels, dem die höchste Menge an wässriger Lösung gemäß Schritt (d) kumulativ zumischbar ist bevor der Wirkstoff nicht mehr vollständig gelöst vorliegt.

Nach einer zweckmäßigen Ausführungsform der Erfindung wird das Verfahren zur Ermittlung des organischen Lösungsmittels mit den organischen Lösungsmitteln Methanol, Ethanol, Isopropanol, n-Butanol, tert.-Butanol, Aceton, Dimethylformamid, Tetrahydrofuran und Dimethylsulfoxid. Gegenstand der Erfindung ist daher auch eine Ausführungsform des erfindungsgemäßen Verfahrens, das dadurch gekennzeichnet ist, dass als organische Lösungsmittel Methanol, Ethanol, Isopropanol, n-Butanol, tert.-Butanol, Aceton, Dimethylformamid, Tetrahydrofuran und Dimethylsulfoxid eingesetzt werden.

Wird in Schritt (b) nach Anspruch 1 (Mischen der organischen und wässrigen Phase) eine zu hohe Menge an wässriger Phase eingesetzt, kann es aufgrund der hiermit einhergehenden Verminderung der Löslichkeit von Wirkstoff und Polymer zu einer Präzipitation des Wirkstoffs kommen, der dann nicht mehr für die Einbettung in die Nanopartikel zur Verfügung steht. Nach einer vorteilhaften Ausbildung der Erfindung wird die Menge an wässriger Phase daher so gewählt, dass nach Mischen der organischen und wässrigen Phase gemäß Schritt (b) von Anspruch 1 die wässrige Phase im Verhältnis zu der organischen Phase in einer Menge vorliegt, die unterhalb der Menge liegt, die der organischen Phase maximal zumischbar ist, ohne dass der Wirkstoff nicht mehr vollständig gelöst vorliegt. Gegenstand der Erfindung ist daher auch eine Ausführungsform des Verfahrens, die dadurch gekennzeichnet ist, dass die Menge an wässriger Phase so gewählt ist, dass nach Mischen der organischen und wässrigen Phase gemäß Schritt (b) von Anspruch 1 die wässrige Phase im Verhältnis zu der organischen Phase in einer Menge vorliegt, die unterhalb der Menge liegt, die der organischen Phase maximal zumischbar ist, ohne dass der Wirkstoff nicht mehr vollständig gelöst vorliegt.

Um sicherzustellen, dass der Wirkstoff vor Beginn der Evaporation vollständig gelöst vorliegt, ist es sinnvoll in dem Verfahren die Menge an wässriger Phase im Verhältnis zu der organischen Phase so zu wählen, dass sie deutlich unterhalb der Menge liegt, die der organischen Phase maximal zumischbar ist, ohne dass der Wirkstoff nicht mehr vollständig gelöst vorliegt. Führt zum Beispiel ein Gemisch aus organischer und wässriger Phase ab einem Volumenverhältnis 3:2 v/v zu Wirkstoffpräzipitation können organische und wässrige Phase in der Colösungsmittelevaporation beispielsweise in einem Volumenverhältnis von 4:1 v/v eingesetzt werden, so dass sichergestellt ist, dass beide Komponenten vollständig gelöst vorliegen.

Bevorzugt ist, dass die Bestimmung der der organischen Phase maximal zumischbaren Menge an wässriger Phase gemäß den Schritten (a) bis (d) des auf Seite 17 beschriebenen Verfahrens für die Ermittlung des organischen Lösungsmittels erfolgt. Gegenstand der Erfindung ist daher auch eine Ausführungsform, die dadurch gekennzeichnet ist, dass die Bestimmung der der organischen Phase maximal zumischbaren Menge an wässriger Phase gemäß den Schritten (a) bis (d) des auf Seite 17 beschriebenen Verfahrenes für die Ermittlung des organischen Lösungsmittels erfolgt.

Die Durchführung des erfindungsgemäßen Verfahrens führt vorteilhaft zu Nanopartikeln mit erhöhter Wirkstoffbeladung und biphasischer Wirkstofffreisetzung, wobei nach Verabreichung zunächst eine schnelle Wirkstofffreisetzung (Initialdosis), die von einer über eine längere Zeit andauernde Wirkstofffreisetzung gefolgt ist. Gegenstand der Erfindung sind daher auch Nanopartikel, die dadurch gekennzeichnet sind, dass sie gemäß dem erfindungsgemäßen Verfahren hergestellt wurden.

Die Beispiele, ohne hierauf beschränkt zu sein, erläutern die Erfindung.

**Beispiele**

**Beispiel 1**

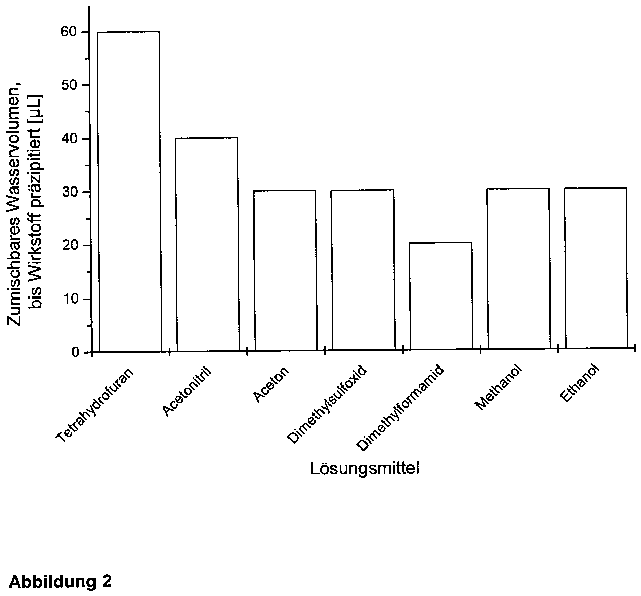

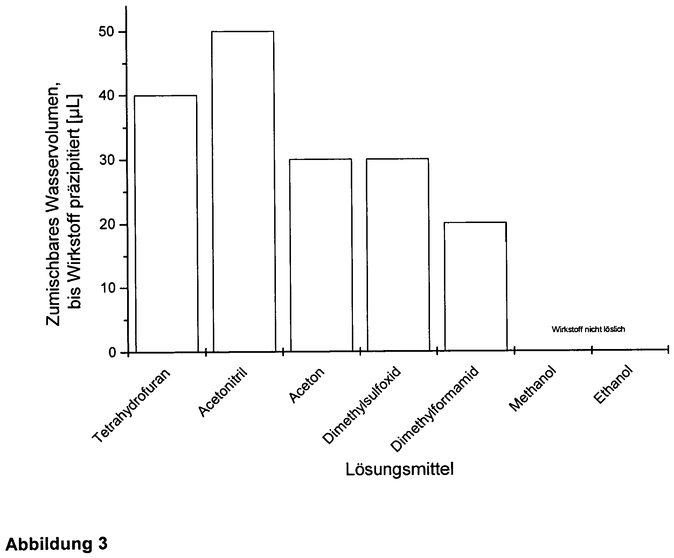

Für Beladungsexperimente wurden die Wirkstoffe Dexamethason und 5-[2-(2-Flouro-phenyl)-[1,8]naphthyridin-4-yl]-[2,6]naphthyridin-1-ylamin (nachfolgend auch Wirkstoff B genannt) verwendet. Zur Lösungsmittelauswahl wurden die Wirkstoffe jeweils mit einer Konzentration von 1 mg/100 μL in folgenden Lösungsmitteln gelöst: Tetrahydrofuran (THF), Acetonitril (ACN), Aceton, Dimethylsulfoxid (DMSO), Dimethylformamid (DMF), Methanol, Ethanol. Jeder organischen Lösung von Wirkstoff B wurden zusätzlich 0.1% Trifluoressigsäure (v/v) zugesetzt, um einen scheinbaren "pH-Wert" einzustellen. Die Lösungen wurden jeweils nacheinander solange mit 10 μL Wasser versetzt und gemischt, bis der Wirkstoff begann zu präzipitieren (optische Löslichkeit). Abbildung 2 zeigt die optische Löslichkeit von Dexamethason, Abbildung 3 die optische Löslichkeit von Wirkstoff B.

Für Dexamethason wurde aufgrund seiner erhöhten optischen Löslichkeit in Tetrahydrofuran dieses Lösungsmittel als organisches Lösungsmittel für die Herstellung der Nanopartikel ausgewählt. Als Startverhältnis wurde 4:1 v/v (THF:Wasser) festgesetzt.

Für Wirkstoff B wurde aufgrund seiner erhöhten optischen Löslichkeit in dieses Lösungsmittel als organisches Lösungsmittel für die Herstellung der Nanopartikel ausgewählt. Als Startverhältnis wurde 5:1 v/v (ACN:Wasser) festgesetzt.

**Beispiel 2**

Zur Herstellung und Beladung von Nanopartikeln wurden folgende Polymere verwendet: PEG-PDLLA [5-b-23], PEG-PCL [5-b-32.5], PEG-PVPy [5-b-20] von Polymersource Inc., Montreal, Canada. Des Weiteren wurde PEG-PLGA [5-b-28] (Resomer RGP 50155 d) von Boehringer Ingelheim, Ingelheim, Deutschland, verwendet. Alle Polymere lagen in Forschungsqualität vor.

Um die andere Herstellungsverfahren mit der Co-Lösungsmittel-Evaporation sowie die Verkapselung in verschiedene Polymere miteinander vergleichen zu können, wurden die Nanopartikel folgendermaßen hergestellt und mit Dexamethason beladen:

- Direkte Dialyse aus Aceton:
  10 mg oder 20 mg Block Copolymer sowie 1 mg bzw. 2 mg Dexamethason wurden in 1 mL Aceton gelöst. Diese Lösung wurde in einen Dialyseschlauch gefüllt (MWCO 6-8 kDa, Spectrumlabs Inc., Breda, Niederlande) und verschlossen. Die Dialyse wurde über 24 h gegen 5 L Wasser ausgeführt; das Wasser wurde ein Mal nach 4 h getauscht. Die entstandene Formulierung wurde anschließend aus dem Dialyseschlauch entnommen, durch einen 0.2 μm Filter gegeben und auf ein Volumen von 2 mL eingestellt.

- O/W Emulsion:
  Vorgeformte Mizellen ohne Wirkstoff wurden zuerst hergestellt wie unter 2.a."Direkte Dialyse" beschrieben. Für die Wirkstoffbeladung wurden 2 mg Dexamethason in 1mL Dichlormethan (VWR, Darmstadt, Deutschland) gelöst. Diese organische Lösung wurde unter stetigem Rühren in 5 mL der wässrigen mizellaren Phase injiziert. Es entstand eine O/W-Emulsion, die über Nacht bei Raumtemperatur weitergerührt wurde. Anschließend erfolgte der Filtrationsschritt durch einen 0.2 μm Filter und die Volumenanpassung auf 5 mL.

- Colösungsmittelevaporation mit anschließender Dialyse:
  10 mg Blockcopolymer und 2 mg Dexamethason wurden in 6 mL THF gelöst. Dieser Lösung wurden 2 mL Wasser hinzugesetzt. Diese Lösung wurde in einem Rundkolben bei einer Temperatur von bei 25°C und einem Druck von 30 mbar über 10 min evaporiert. Die erhaltene Formulierung wurde in einen Float-A-Lyzer G2 Dialyseschlauch (MWCO 8-10 kDa, Spectrumlabs Inc., Berda, Niederlande) gegeben, der im Fall der anschließenden Dialyse gegen gesättigte Lösung in Dexamethason-gesättigtem Wasser vorequilibriert worden war. Die Formulierung wurde sodann gegen 5 L Wasser oder Dexamethason-gesättigtem Wasser für 24 h dialysiert. Abschließend wurde die Formulierung durch einen 0.2 μm Filter gegeben und das Volumen auf 2 mL eingestellt.

Die nach den verschiedenen Verfahren hergestellten Nanopartikel wurden hinsichtlich ihrer Wirkstoffbeladung und Partikelgrößen und deren Größenverteilungen charakterisiert.

Bestimmung der Wirkstoffbeladuna über HPLC

100 μL der resultierenden mizellaren Formulierung wurden in 900 μL Acetonitril gelöst. Diese Lösung wurde mit einem HPLC System (Merck Hitachi La Chrom Elite) über einen UV-Detektor (Detektionswellenlange:282 nm) detektiert. Die Trennung erfolgte auf einer Agilent Eclipse Plus C18 Säule (3.5 μm Körnung, 5 cm Länge) bei 30°C. Eine Gradientenmethode wurde für die Trennung genutzt. Dabei bestand die mobile Phase A aus 90% Acetronitril und 10% Ammoniumacetatpuffer pH 4.5 (v/v), die mobile Phase B hatte die umgekehrte Zusammensetzung. Die Dexamethason Probenkonzentration wurde über eine Kalibierkurve ermittelt.

Die Berechnung der Wirkstoffbeladung erfolgte über folgende Formel 1:

$$\text{Wirkstoffbeladung [\%]} = \frac{\text{Wirkstoffkonzentration}\left[\frac{mg}{mL}\right]}{\text{Polymerkonzentration}\left[\frac{mg}{mL}\right]} \cdot 100\% \qquad (1)$$

Partikelarößenbestimmung mittels Dynamischer Lichtstreuung (DLS)

Die DLS-Technik ermittelt den hydrodynamischen Partikelradius bzw. - durchmesser. Dafür werden die Proben 1:100 (v/v) mit Wasser verdünnt und in einem Malvern Zetasizer Nano ZS (Malvern Instruments Ltd., Worcestershire, UK) im Rückstreumodus vermessen. Partikelgrößen wurden über die Kumulantenanalyse berechnet. Zusätzlich wurde der Polydispersitätsindex (PdI, polydispersity index) berechnet, der als Maß für die Streuung der Partikelgrößenverteilung gilt. Der PdI kann Werte zwischen 0 und 1 aufweisen wobei 0 monodispers und 1 (völlig) polydispers bedeutet.

Die Ergebnisse sind in nachfolgender Tabelle 1 zusammengestellt.

| Polymer | Polymer Konzentration [%] w/v | Wirkstoff/ Polymer Verhältnis initial | Herstellungs-methode | Lösungsmittel und Bedingungen | Wirkstoff-beladung [%] | Hydrod-ynamische Partikelgröße [nm] | PdI |
|---|---|---|---|---|---|---|---|
| PEG-PDLLA [5-b-23] | 0.5 | 1:5 | Co-LM evapo-ration | THF, Dialyse gegen Wasser | < LOQ | 50.41 ± 2.47 | 0.120 ± 0.047 |
| PEG-PDLLA [5-b-23] | 0.5 | 1:5 | Co-LM evapo-ration | THF, Dialyse gegen Wirkstoff gesättigte Lsg. | 1.56 ± 0.24 | 61.43 ± 1.39 | 0.102 ± 0.006 |
| PEG-PLGA [5-b-28] | 0.5 | 1:5 | Co-LM evapo-ration | THF, Dialyse gegen Wasser | < LOQ | 62.67 ± 1.60 | 0.091 ± 0.014 |
| PEG-PLGA [5-b-28] | 0.5 | 1:5 | Co-LM evapo-ration | THF, Dialyse gegen Wirkstoff gesättigte Lsg | 1.19 ± 0.13 | 69.18 ± 1.23 | 0.057 ± 0.026 |
| PEG-PCL [5-b-32.5] | 0.5 | 1:5 | Co-LM evapo-ration | THF, Dialyse gegen Wasser | < LOQ | 80.59 ± 2.98 | 0.093 ± 0.053 |
| PEG-PCL [5-b-32.5] | 0.5 | 1:5 | Co-LM evapo-ration | THF, Dialyse gegen Wirkstoff gesättigte Lsg | 1.39 ± 0.36 | 87.69 ± 2.70 | 0.126 ± 0.034 |
| PEG-PVPy [5-b-20] | 0.5 | 1:5 | Co-LM evapo-ration | THF, Dialyse gegen Wasser | 10.74 ± 1.8 | 33.97 ± 1.50 | 0.204 ± 0.016 |
| PEG-PVPy [5-b-20] | 0.5 | 1:5 | Co-LM evapo-ration | THF, Dialyse gegen Wirkstoff gesättigte Lsg | 18.67 ± 0.21 | 36.73 ± 0.95 | 0.213 ± 0.006 |

| PEG-PVPy [5-b-20] | 1.0 | 1:5 | Co-LM evapo-ration | THF, Dialyse gegen **Wirkstoff gesättigte Lsg** | 19.25 ± 0.54 | 52.13 ± 1.34 | 0.258 ± 0.011 |
|---|---|---|---|---|---|---|---|
| PEG-PVPy [5-b-20] | 0.5 | 1:5 | Co-LM evapo-ration | Aceton, Dialyse gegen **Wirkstoff gesättigte Lsg.** | 12.07 ± 1.21 | 41.09 ± 2.80 | 0.136 ± 0.011 |
| PEG-PVPy [5-b-20] | 1.0 | 1:5 | Co-LM evapo-ration | Aceton, Dialyse gegen **Wirkstoff gesättigte Lsg.** | 10.84 ± 2.64 | 44.73 ± 4.65 | 0.118 ± 0.011 |
| PEG-PVPy [5-b-20] | 0.5 | 1:10 | Direkte Dialyse | Aceton | 1.71 ± 0.15 | 56.42 ± 7.29 | 0.178 ± 0.056 |
| PEG-PVPy [5-b-20] | 1.0 | 1:5 | Direkte Dialyse | Aceton | 0.62 ± 0.60 | 66.91 ± 2.29 | 0.162 ± 0.011 |
| PEG-PVPy [5-b-20] | 0.5 | 1:10 | O/W Emul-sion | Dichlor-methan, vorgefertige Partikel aus Aceton | 8.74 ± 0.03 | 52.42 ± 2.00 | 0.150 ± 0.012 |
| PEG-PVPy [5-b-20] | 1.0 | 1:10 | O/W Emul-sion | Dichlor-methan, vorgefertige Partikel aus Aceton | 7.81 ± 0.18 | 68.92 ± 3.53 | 0.185 ± 0.036 |
| PEG-PVPy [5-b-20] | 0.5 | 2:5 | O/W Emul-sion | Dichlormethan, vorgefertige Partikel aus Aceton | 13.50 ± 5.05 | 52.19 ± 0.67 | 0.186 ± 0.022 |

Tabelle 1, worin PEG-PDLLA pegylierte Poly-(D,L-milchsäure), PEG-PLGA pegylierte Poly-(milchsäure-co-glykolsäure), PEG-PCL pegyliertes Poly-

(caprolacton), PEG-PVPy pegyliertes Poly-4-(vinylpyridine), LOQ Limit of quantification (Bestimmungsgrenze der HPLC-Methode), Co-LM evaporation Colösungsmittelevaporation und THF Tetrahydrofuran bedeutet.

**Beispiel 3**

Herstellung und Beladung von mit Wirkstoff B beladenen Nanopartikeln

Die Herstellung und Beladung der Nanopartikel erfolgte nach dem in dieser Erfindung beschriebenen Verfahren als Kombination zwischen Colösungsmittelevaporation sowie Dialyse gegen wirkstoffgesättigte Lösung.

10 mg Blockcopolymer und 1 mg Wirkstoff wurden in 8 mL Acetonitril/0.1% Trifluoressigsäure (v/v) unter Ultraschallbehandlung gelöst. Die erhaltene Lösung wurde mit 2 mL Wasser gemischt. Anschließend wurde die Mischung in einen Rundkolben gegeben und das organische Lösungsmittel bei Unterdruck (30 mbar) und 25°C evaporiert (10 min). Die erhaltenen Nanopartikel wurden in einen Float-A-Lyzer G2 Dialyseschlauch (MWCO 8-10 kDa, Spectrumlabs Inc., Berda, Niederlande) gegeben und 24 h gegen 5 L mit Wirkstoff B gesättigter phosphatgepufferter Kochsalzlösung (PBS Puffer) pH 7.4 dialysiert. Zum Abschluss wurde die Formulierung durch einen 0.2 $\mu$m Filter gegeben und das Volumen der Formulierung auf 2 mL eingestellt.

Bestimmung der Wirkstoffbeladung

100 $\mu$L der erhaltenen Formulierung wurden in 900 $\mu$L Acetonitril gelöst. Diese Lösung wurde mit einem HPLC System (Merck Hitachi La Chrom Elite) über einen UV-Detektor (Detektionswellenlange:254 nm) detektiert. Die Trennung erfolgte auf einer Agilent Eclipse Plus C18 Säule (3.5 $\mu$m Körnung, 5 cm Länge) bei 30°C. Eine Gradientenmethode wurde für die Trennung genutzt. Dabei bestand die mobile Phase A aus 90% Acetonitril und 10% Wasser mit 0.1% Trifluoressigsäure (v/v), die mobile Phase B hatte die umgekehrte Zusammensetzung. Die Wirkstoff B Probenkonzentration wurde über eine Kalibrierkurve ermittelt.

Die Berechnung der Wirkstoffbeladung erfolgte hierbei gemäß Formel 1. Partikelgrößen sowie Größenverteilungen wurden analog Beispiel 2 bestimmt.

Die Ergebnisse mit der optimalen Beladungstechnik bei Verwendung verschiedener Blockcopolymere sind in Tabelle 2 zusammengestellt.

| Polymer | Polymer Konzentration [%] w/v | Wirkstoff/ Polymer Verhältnis initial | Herstellungs- methode | Lösungsmittel und Bedingungen | Wirkstoff- beladung [%] | Hydrod- ynamische | Pdl |
|---|---|---|---|---|---|---|---|
| PEG-PDLLA [5-b-23] | 0.5 | 1:10 | Co-LM evaporation | ACN/0.1% TFA, Dialyse mit Sättigung | < LOQ | n.a. | n.a. |
| PEG-PLGA [5-b-28] | 0.5 | 1:10 | Co-LM evaporation | ACN/0.1% TFA, Dialyse mit Sättigung | 25.4 | n.a. | n.a. |
| PEG-PCL [5-b-32.5] | 0.5 | 1:10 | Co-LM evaporation | ACN/0.1% TFA, Dialyse mit Sättigung | 24.9 | n.a. | n.a. |
| PEG-PVPy [5-b-20] | 0.5 | 1:10 | Co-LM evaporation | ACN/0.1% TFA, Dialyse mit Sättigung | 40.6 | n.a. | n.a. |
| PEG-PLGA [5-b-28] | 5.0 | 1:10 | Co-LM evaporation | ACN/0.1% TFA, Dialyse mit Sättigung | 101.90 ± 6.44 | 122 | 0,183 |

Tabelle 2, worin PEG-PDLLA pegylierte Poly-(D,L-milchsäure), PEG-PLGA pegylierte Poly-(milchsäure-co-glykolsäure), PEG-PCL pegyliertes Poly-(caprolacton), PEG-PVPy pegyliertes Poly-4-(vinylpyridine), LOQ Limit of quantification (Bestimmungsgrenze der HPLC-Methode), Co-LM evaporation Colösungsmittelevaporation, ACN Acetonitril und TFA Trifluoressigsäure bedeutet.

**Patentansprüche**

1. Verfahren zur Herstellung von Nanopartikeln umfassend die Schritte

(a) Lösen von mindestens einem Wirkstoff und mindestens einem Polymer in einem organischen Lösungsmittel,
(b) Mischen der in Schritt (a) hergestellten Lösung mit einer wässrigen Phase,
(c) Evaporieren des organischen Lösungsmittels,
(d) Aufreinigen der in Schritt (c) erhaltenen, mit Wirkstoff beladenen Nanopartikel mittels Dialyse gegen den gleichen Wirkstoff enthaltende wässrige Dialyselösung.

2. Verfahren nach Anspruch 1 und/oder 2, **dadurch gekennzeichnet, dass** der Wirkstoff in Wasser eine Sättigungs-löslichkeit < 200 $\mu$g/ml, bevorzugt eine Sättigungslöslichkeit <100 $\mu$g/ml, jeweils gemessen bei 25°C, aufweist.

3. Verfahren nach Anspruch 2, **dadurch gekennzeichnet, dass** als Wirkstoff ein Wirkstoff verwendet wird, das ausgewählt ist aus der Gruppe bestehend aus Chemotherapeutika, insbesondere Taxol, Camptothecin, Platinkomplexe oder N-Lost-Verbindungen, Antirheumatika, wie zum Beispiel Glucocorticoide, insbesondere Dexamethason, Mometason, Beclomethason oder Prednisolon, Antiinfektiva, wie zum Beispiel HIV-Therapeutika, insbesondere Ritonavir, sowie Antimykotika, insbesondere Ketoconazol, Itraconazol, Griseofulvin, Lipidsenker, wie zum Beispiel Fenofibrat, Antioxidatien und Vitamine, wie zum Beispiel Tocopherol-, Retinolsäure-, Cholecalciferol, Antibiotika, wie zum Beispiel Vancomycin oder Teicomycin, zusätzlich Cholesterol sowie Fettsäuren.

**4.** Verfahren nach einem oder mehreren der Ansprüche 1 bis 3, **dadurch gekennzeichnet, dass** als Polymer ein amphiphiles Polymer eingesetzt wird.

**5.** Verfahren nach Anspruch 4, **dadurch gekennzeichnet, dass** als Polymer ein Blockcopolymer eingesetzt wird.

**6.** Verfahren nach Anspruch 4 und/oder 5, **dadurch gekennzeichnet, dass** das Blockcopolymer als hydrophile Komponente Polyethylenglykol, Polypropylenglykol, Polybutylenglykol, Polyacrylamid, Polyvinylalkohol, Polysaccharid oder ein Copolymer hiervon, bevorzugt Polyethylenglykol/Polypropylenglykol-Copolymer, Polyethylenglykol/Polypropylenglykol/Polyethylenglykol-Copolymer, und als hydrophobe Komponente Polymilchsäure, Polyglykolsäure, Polyhydroxybuttersäure, Polyhydroxyvaleriansäure, oder ein Copolymer hiervon, bevorzugt Polymilch-co-glykolsäure, weiterhin Polyacrylsäure , insbesondere Hydroxypropylethylacrylsäure oder Hydroxypropylmethylacrylsäure, Polysiloxan , insbesondere Copolymere mit Acrylsäure, Polystyren oder ein Copolymer hiervon, insbesondere mit Polymilchsäure und Polyglykolsäure enthält.

**7.** Verfahren nach Anspruch 6, **dadurch gekennzeichnet, dass** als Blockcopolymere Polyethylenglykol-Polymilchsäure, Polyethylenglykol-Polyglykolsäure, Polyethylenglykol-Polymilchsäure-co-glykolsäure, Polyethylenglykol-Polyhydroxyvaleriansäure, Polyethylenglykol-Polysiloxan, Polyethylenglykol-Polysiloxan-co-acrylsäure, Polyethylenglykol-Polymethylmethacrylsäure, Polyethylenglykol-Polymethyl-ethacrylsäure, Polyethylenglykol-Polyisoprylacrylsäure, Polyethylenglykol-Polystyren eingesetzt wird.

**8.** Verfahren nach einem oder mehreren der Ansprüche 1 bis 7, **dadurch gekennzeichnet, dass** als organisches Lösungsmittel ein Lösungsmittel verwendet wird, das mit Wasser zumindest teilweise mischbar, bevorzugt vollständig mischbar ist.

**9.** Verfahren nach Anspruch 8, **dadurch gekennzeichnet, dass** als organisches Lösungsmittel lineare oder verzweigtkettige Alkohole, vorzugsweise Methanol, Ethanol, Isopropanol, n-Butanol oder tert.-Butanol, Aceton , Dimethylformamid, Tetrahydrofuran oder Dimethylsulfoxid eingesetzt wird.

**10.** Verfahren nach einem oder mehreren der Ansprüche 1 bis 9, **dadurch gekennzeichnet, dass** in Schritt (a) gemäß Anspruch 1 in dem organischen Lösungsmittel neben Polymer und Wirkstoff eine Säure oder eine Base gelöst ist und/oder dass in dem wässrigen Lösungsmittel gemäß Schritt (b) von Anspruch 1 eine Säure oder eine Base gelöst ist.

**11.** Verfahren nach Anspruch 10, **dadurch gekennzeichnet, dass** die Säure eine organische Säure, vorzugsweise Ameisensäure, Essigsäure oder Trifluoressigsäure, oder eine anorganische Säure, vorzugsweise Salzsäure, Salpetersäure oder Schwefelsäure, und die Base eine organische Base, vorzugsweise Dimethylamin oder Trimethylamin oder eine anorganische Base, vorzugsweise Natriumhydroxid, Kaliumhydroxid oder Ammoniak, ist.

**12.** Verfahren nach einem oder mehreren der Ansprüche 1 bis 11, **dadurch gekennzeichnet, dass** bei der Herstellung der Nanopartikel gemäß Schritt (a) von Anspruch 1 als organisches Lösungsmittel dasjenige organische Lösungsmittel verwendet wird, dem bei Herstellung einer den Wirkstoff in definierter Menge enthaltenden Lösung im Vergleich zu diesen Wirkstoff in jeweils gleicher Menge enthaltenden Lösungen in anderen organischen Lösungsmitteln bei sukzessiver Zumischung von wässrigem Lösungsmittel der höchste Anteil an wässrigem Lösungsmittel zumischbar ist, ohne dass dieser aus der Lösung ausfällt.

**13.** Verfahren nach Anspruch 12, **dadurch gekennzeichnet, dass** das organische Lösungsmittel gemäß nachfolgendem Verfahren ermittelt wird:

(a) Herstellen von Lösungen des Wirkstoffs mit jeweils gleichem Wirkstoffanteil in verschiedenen organischen Lösungsmitteln,
(b) Zugeben einer jeweils gleichen Menge wässriger Lösung zu jeder der in Schritt (a) hergestellten Lösungen,
(c) Überprüfen ob der Wirkstoff in den Lösungen von Schritt (b) jeweils vollständig gelöst vorliegt,
(d) wiederholtes Durchführen der Schritte (b) und (c) mit den Lösungen, in denen der Wirkstoff gemäß Schritt (c) vollständig gelöst vorliegt, bis der Wirkstoff gemäß Schritt (c) nicht mehr vollständig gelöst vorliegt,
(e) Identifizieren des organischen Lösungsmittels, dem die höchste Menge an wässriger Lösung gemäß Schritt (d) kumulativ zumischbar ist bevor der Wirkstoff nicht mehr vollständig gelöst vorliegt.

**14.** Verfahren gemäß Anspruch 12 und/oder 13, **dadurch gekennzeichnet, dass** als organische Lösungsmittel Methanol, Ethanol, Isopropanol, n-Butanol, tert.-Butanol, Aceton, Dimethylformamid, Tetrahydrofuran und Dimethylsulfoxid eingesetzt werden.

15. Verfahren nach einem oder mehreren der Ansprüche 1 bis 14, **dadurch gekennzeichnet, dass** die Menge an wässriger Phase so gewählt ist, dass nach Mischen der organischen und wässrigen Phase gemäß Schritt (b) die wässrige Phase im Verhältnis zu der organischen Phase in einer Menge vorliegt, die unterhalb der Menge liegt, die der organischen Phase maximal zumischbar ist, ohne dass der Wirkstoff nicht mehr vollständig gelöst vorliegt.

16. Verfahren gemäß Anspruch 15, **dadurch gekennzeichnet, dass** die Bestimmung der der organischen Phase maximal zumischbaren Menge an wässriger Phase gemäß den Schritten (a) bis (d) von Anspruch 13 erfolgt.

**Claims**

1. Process for the production of nanoparticles comprising the steps of

   (a) dissolution of at least one active compound and at least one polymer in an organic solvent,
   (b) mixing of the solution prepared in step (a) with an aqueous phase,
   (c) evaporation of the organic solvent,
   (d) purification of the nanoparticles laden with active compound obtained in step (c) by means of dialysis against aqueous dialysis solution comprising the same active compound.

2. Process according to Claim 1 and/or 2, **characterised in that** the active compound has a saturation solubility in water < 200 $\mu$g/ml, preferably a saturation solubility < 100 $\mu$g/ml, in each case measured at 25°C.

3. Process according to Claim 2, **characterised in that** the active compound used is an active compound which is selected from the group consisting of chemotherapeutic agents, in particular taxol, camptothecin, platinum complexes or N-mustard compounds, antirheumatics, such as, for example, glucocorticoids, in particular dexamethasone, mometasone, beclomethasone or prednisolone, anti-infective agents, such as, for example, HIV therapeutic agents, in particular ritonavir, and antimycotic agents, in particular ketoconazole, itraconazole, griseofulvin, lipid-lowering agents, such as, for example, fenofibrate, antioxidants and vitamins, such as, for example, tocopherol, retinoic acid, cholecalciferol, antibiotics, such as, for example, vancomycin or teicomycin, additionally cholesterol and fatty acids.

4. Process according to one or more of Claims 1 to 3, **characterised in that** the polymer employed is an amphiphilic polymer.

5. Process according to Claim 4, **characterised in that** the polymer employed is a block copolymer.

6. Process according to Claim 4 and/or 5, **characterised in that** the block copolymer contains as hydrophilic component polyethylene glycol, polypropylene glycol, polybutylene glycol, polyacrylamide, polyvinyl alcohol, polysaccharide or a copolymer thereof, preferably polyethylene glycol-polypropylene glycol copolymer, polyethylene glycol-polypropylene glycol-polyethylene glycol copolymer, and as hydrophobic component polylactic acid, polyglycolic acid, polyhydroxybutyric acid, polyhydroxyvaleric acid, or a copolymer thereof, preferably polylactic-co-glycolic acid, furthermore polyacrylic acid, in particular hydroxypropylethylacrylic acid or hydroxypropylmethylacrylic acid, polysiloxane, in particular copolymers with acrylic acid, polystyrene or a copolymer thereof, in particular with polylactic acid and polyglycolic acid.

7. Process according to Claim 6, **characterised in that** the block copolymers employed is polyethylene glycol-polylactic acid, polyethylene glycol-polyglycolic acid, polyethylene glycol-polylactic acid-co-glycolic acid, polyethylene glycol-polyhydroxyvaleric acid, polyethylene glycol-polysiloxane, polyethylene glycol-polysiloxane-co-acrylic acid, polyethylene glycol-poly-methylmethacrylic acid, polyethylene glycol-polymethylethacrylic acid, polyethylene glycol-polyisoprylacrylic acid, polyethylene glycol-polystyrene.

8. Process according to one or more of Claims 1 to 7, **characterised in that** the organic solvent used is a solvent which is at least partially miscible, preferably fully miscible, with water.

9. Process according to Claim 8, **characterised in that** the organic solvent employed is linear or branched-chain alcohols, preferably methanol, ethanol, isopropanol, n-butanol or tert-butanol, acetone, dimethylformamide, tetrahydrofuran or dimethyl sulfoxide.

10. Process according to one or more of Claims 1 to 9, **characterised in that**, in step (a) according to Claim 1, an acid or base is dissolved in the organic solvent besides polymer and active compound, and/or **in that** an acid or base is dissolved in the aqueous solvent in step (b) of Claim 1.

11. Process according to Claim 10, **characterised in that** the acid is an organic acid, preferably formic acid, acetic acid or trifluoroacetic acid, or an inorganic acid, preferably hydrochloric acid, nitric acid or sulfuric acid, and the base is an organic base, preferably dimethylamine or trimethylamine, or an inorganic base, preferably sodium hydroxide, potassium hydroxide or ammonia.

12. Process according to one or more of Claims 1 to 11, **characterised in that** the organic solvent used in the production of the nanoparticles in step (a) of Claim 1 is the organic solvent with which the greatest proportion of aqueous solvent can be admixed without this precipitating out of the solution during preparation of a solution comprising the active compound in defined amount compared with solutions comprising this active compound in the same amount in each case in other organic solvents on successive admixing of aqueous solvent.

13. Process according to Claim 12, **characterised in that** the organic solvent is determined by the following method:

(a) preparation of solutions of the active compound having the same proportion of active compound in each case in various organic solvents,
(b) addition of an in each case identical amount of aqueous solution to each of the solutions prepared in step (a),
(c) checking whether the active compound is in each case fully dissolved in the solutions of step (b),
(d) repeated performance of steps (b) and (c) with the solutions in which the active compound is fully dissolved in step (c), until the active compound is no longer fully dissolved in step (c),
(e) identification of the organic solvent with which the greatest amount of aqueous solution can be admixed cumulatively in step (d) before the active compound is no longer fully dissolved.

14. Process according to Claim 12 and/or 13, **characterised in that** the organic solvents employed are methanol, ethanol, isopropanol, n-butanol, tert-butanol, acetone, dimethylformamide, tetrahydrofuran and dimethyl sulfoxide.

15. Process according to one or more of Claims 1 to 14, **characterised in that** the amount of aqueous phase is selected so that, after mixing of the organic and aqueous phase in step (b), the aqueous phase is present in an amount, in relation to the organic phase, which is below the maximum amount which can be admixed with the organic phase without the active compound no longer being fully dissolved.

16. Process according to Claim 15, **characterised in that** the determination of the maximum amount of aqueous phase which can be admixed with the organic phase is carried out in accordance with steps (a) to (d) of Claim 13.

**Revendications**

1. Procédé de production de nanoparticules, comprenant les étapes

(a) de dissolution d'au moins un ingrédient actif et d'au moins un polymère dans un solvant organique,
(b) de mélange de la solution préparée dans l'étape (a) avec une phase aqueuse,
(c) d'évaporation du solvant organique,
(d) de purification des nanoparticules chargées avec l'ingrédient actif obtenues dans l'étape (c) au moyen d'une dialyse contre une solution de dialyse aqueuse comprenant le même ingrédient actif.

2. Procédé selon la revendication 1 et/ou 2, **caractérisé en ce que** l'ingrédient actif possède une solubilité à saturation dans l'eau < 200 $\mu$g/ml, préférablement une solubilité à saturation < 100 $\mu$g/ml, dans chaque cas mesurée à 25°C.

3. Procédé selon la revendication 2, **caractérisé en ce que** l'ingrédient actif utilisé est un ingrédient actif qui est choisi dans le groupe constitué par les agents chimiothérapeutiques, en particulier le taxol, la camptothécine, les complexes de platine ou les composés de N-moutarde, les agents anti-rhumatismaux, tels que par exemple les glucocorticoïdes, en particulier la dexaméthasone, la mométasone, la béclométhasone ou la prednisolone, les agents anti-infectieux, tels que par exemple les agents thérapeutiques anti-VIH, en particulier le ritonavir, et les agents antimycotiques,

EP 2 819 659 B1

en particulier le kétoconazole, l'itraconazole, la griséofulvine, les agents hypolipidémiants, tels que par exemple le fénofibrate, les antioxydants et les vitamines, tels que par exemple le tocophérol, l'acide rétinoïque, le cholécalciférol, les antibiotiques, tels que par exemple la vancomycine ou la teïcomycine, en outre le cholestérol et les acides gras.

**4.** Procédé selon l'une ou plusieurs parmi les revendications 1 à 3, **caractérisé en ce que** le polymère employé est un polymère amphiphile.

**5.** Procédé selon la revendication 4, **caractérisé en ce que** le polymère employé est un polymère bloc.

**6.** Procédé selon la revendication 4 et/ou 5, **caractérisé en ce que** le copolymère bloc contient, comme composant hydrophile, du polyéthylène glycol, du polypropylène glycol, du polybutylène glycol, du polyacrylamide, de l'alcool polyvinylique, un polysaccharide ou un copolymère de ceux-ci, préférablement un copolymère de polyéthylène glycol-polypropylène glycol, un copolymère de polyéthylène glycol-polypropylène glycol-polyéthylène glycol, et, comme composant hydrophobe, de l'acide polylactique, de l'acide polyglycolique, de l'acide polyhydroxybutyrique, de l'acide polyhydroxyvalérique, ou un copolymère de ceux-ci, préférablement l'acide polylactique-co-glycolique, en outre de l'acide polyacrylique, en particulier l'acide hydroxypropyléthylacrylique ou l'acide hydroxypropyléthylacrylique, un polysiloxane, en particulier des copolymères avec l'acide acrylique, du polystyrène ou un copolymère de celui-ci, en particulier avec l'acide polylactique et l'acide polyglycolique.

**7.** Procédé selon la revendication 6, **caractérisé en ce que** les copolymères blocs employés sont le polyéthylène glycol-acide polylactique, le polyéthylène glycol-acide polyglycolique, le polyéthylène glycol-acide polylactique-co-acide glycolique, le polyéthylène glycol-acide polyhydroxyvalérique, le polyéthylène glycol-polysiloxane, le polyéthylène glycol-polysiloxane-co-acide acrylique, le polyéthylène glycol-acide polyméthylméthacrylique, le polyéthylène glycol-acide polyméthyléthacrylique, le polyéthylène glycol-acide polyisopropylacrylique, le polyéthylène glycol-polystyrène.

**8.** Procédé selon l'une ou plusieurs parmi les revendications 1 à 7, **caractérisé en ce que** le solvant organique utilisé est un solvant qui est au moins partiellement miscible, préférablement totalement miscible, avec l'eau.

**9.** Procédé selon la revendication 8, **caractérisé en ce que** le solvant organique employé est constitué d'alcools à chaîne linéaire ou ramifiée, préférablement le méthanol, l'éthanol, l'isopropanol, le n-butanol ou le tertio-butanol, l'acétone, le diméthylformamide, le tétrahydrofurane ou le diméthylsulfoxyde.

**10.** Procédé selon l'une ou plusieurs parmi les revendications 1 à 9, **caractérisé en ce que**, dans l'étape (a) selon la revendication 1, un acide ou une base est solubilisé(e) dans le solvant organique outre le polymère et l'ingrédient actif, et/ou **en ce qu'**un acide ou une base est solubilisé(e) dans le solvant aqueux dans l'étape (b) selon la revendication 1.

**11.** Procédé selon la revendication 10, **caractérisé en ce que** l'acide est un acide organique, préférablement l'acide formique, l'acide acétique ou l'acide trifluoroacétique, ou un acide inorganique, préférablement l'acide chlorhydrique, l'acide nitrique ou l'acide sulfurique, et la base est une base organique, préférablement la diméthylamine ou la triméthylamine, ou une base inorganique, préférablement l'hydroxyde de sodium, l'hydroxyde de potassium ou l'ammoniaque.

**12.** Procédé selon l'une ou plusieurs parmi les revendications 1 à 11, **caractérisé en ce que** le solvant organique utilisé dans la production des nanoparticules dans l'étape (a) selon la revendication 1 est le solvant organique avec lequel la plus grande proportion de solvant aqueux peut être mélangée lors de la préparation d'une solution comprenant l'ingrédient actif en quantité définie sans que ce dernier ne précipite dans la solution par rapport à des solutions comprenant cet ingrédient actif selon la même quantité dans chaque cas dans d'autres solvants organiques lors d'un mélange successif de solvant aqueux.

**13.** Procédé selon la revendication 12, **caractérisé en ce que** le solvant organique est déterminé par la méthode suivante :

(a) la préparation de solutions de l'ingrédient actif ayant la même proportion d'ingrédient actif dans chaque cas dans divers solvants organiques,
(b) l'addition d'une quantité dans chaque cas identique de solution aqueuse à chacune des solutions préparées dans l'étape (a),

(c) la vérification du fait que l'ingrédient actif est, ou non, dans chaque cas totalement solubilisé dans les solutions de l'étape (b),

(d) la mise en oeuvre répétée des étapes (b) et (c) avec les solutions dans lesquelles l'ingrédient actif est totalement solubilisé dans l'étape (c), jusqu'à ce que l'ingrédient actif ne soit plus totalement solubilisé dans l'étape (c),

(e) l'identification du solvant organique avec lequel la plus grande quantité de solution aqueuse peut être mélangée de manière cumulée dans l'étape (d) avant que l'ingrédient actif ne soit plus totalement solubilisé.

14. Procédé selon la revendication 12 et/ou 13, **caractérisé en ce que** les solvants organiques employées sont le méthanol, l'éthanol, l'isopropanol, le n-butanol, le tertio-butanol, l'acétone, le diméthylformamide, le tétrahydrofurane et le diméthylsulfoxyde.

15. Procédé selon l'une ou plusieurs parmi les revendications 1 à 14, **caractérisé en ce que** la quantité de phase aqueuse est choisie de sorte que, après mélange de la phase organique et aqueuse dans l'étape (b), la phase aqueuse soit présente selon une quantité, par rapport à la phase organique, qui est inférieure à la quantité maximale pouvant être mélangée avec la phase organique sans que l'ingrédient actif ne soit plus totalement solubilisé.

16. Procédé selon la revendication 15, **caractérisé en ce que** la détermination de la quantité maximale de phase aqueuse pouvant être mélangée avec la phase organique est effectuée conformément aux étapes (a) à (d) selon la revendication 13.

Lösen von 1 mg Wirkstoff/100 µl Lösungsmittel

Klare Lösung → Schrittweise Zugabe von Wasser/Puffer → Auswahl des Lösungsmittels, welches den Wirkstoff am längsten in Lösung hält (optische Löslichkeit)

Wirkstoff nicht löslich → Lösungsmittel als nicht geeignet verworfen

**Abbildung 1**

**Abbildung 2**

**Abbildung 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **BEISPIEL V.P.SANT ; D.SMITH ; J.C.LEROUX.** Enhancement of oral bioavailability of poorly water-soluble drugs by poly(ethylene glycol)-block-poly(alkyl acrylate-co-methacrylic acid) self-assemblies. *J Control Release.,* 2005, vol. 104, 289-300
- **K.AVGOUSTAKIS ; A.BELETSI ; Z.PANAGI ; P.KLEPETSANIS ; A.G.KARYDAS ; D.S.ITHAKISSIOS.** PLGA-mPEG nanoparticles of cisplatin: in vitro nanoparticle degradation, in vitro drug release and in vivo drug residence in blood properties. *J Control Release,* 2002, vol. 79, 123-135
- **C.X.SONG ; V.LABHASETWAR ; H.MURPHY ; X.QU ; W.R.HUMPHREY ; R.J.SHEBUSKI ; R.J.LEVY.** Formulation and characterization of biodegradable nanoparticles for intravascular local drug delivery. *Journal of Controlled Release,* 1997, vol. 43, 197-212
- **H.J.JEON ; J.I.JEONG ; M.K.JANG ; Y.H.PARK ; J.W.NAH.** Effect of solvent on the preparation of surfactant-free poly(DL-lactide-co-glycolide) nanoparticles and norfloxacin release characteristics. *International Journal of Pharmaceutics,* 2000, vol. 207, 99-108
- **A.RICHTER ; C.OLBRICH ; M.KRAUSE ; J.HOFFMANN ; T.KISSEL.** Polymeric micelles for parenteral delivery of Sagopilone: physicochemical characterization, novel formulation approaches and their toxicity assessment in vitro as well as in vivo. *Eur J Pharm Biopharm,* 2010, vol. 75, 80-89

- **E.BLANCO ; E.A.BEY ; Y.DONG ; B.D.WEINBERG ; D.M.SUTTON ; D.A.BOOTHMAN ; J.GAO.** Beta-lapachone-containing PEG-PLA polymer micelles as novel nanotherapeutics against NQO1-overexpressing tumor cells. *J Control Release,* 2007, vol. 122, 365-374
- **K. LECHTFORD et al.** A a review about formation and classification of amphiphilic block copolymer nanoparticulate structures:micelles, nanospheres, nanocapsules and polymersomes. *Eur J Pharm Biopharm,* 2007, vol. 65, 259-269
- **LAMPRECHT A. et al.** beschreibt eine Dialyse von Nanopartikeln gegen reines Wasser. *um Hilfsstoffe und nicht verkapselten Wirkstoff zu entfernen*
- **ALF LAMPRECHT A. et al.** Design of rolipram-loaded nanoparticles: comparison of two preparation methods. *J Contr Release,* 2001, vol. 71, 297-306
- **K. ELKHARRAZ E. et al.** beschreibt die Herstellung von Wirkstoff-freien und mit Paclitaxel beladenen PLGA-Mikropartikeln mit einem Öl. *Wasser (O/W) Lösungsmittel-Extraktions- / Evaporationsmethode*
- Paclitaxel-loaded microparticles and implants for the treatment of brain cancer: Preparation and physicochemical characterization. *Int J Pharm,* 2006, vol. 314, 127-136